# EUROPEAN PATENT APPLICATION

(11) **EP 3 173 790 A1**
(43) Date of publication of application: **31.05.2017**
(21) Application number: 15003344.7
(22) Date of filing: 24.11.2015
(51) Int. Cl.: G01N 33/68

(54) **ALLERGY SCREENING PLATFORM**

(71) Applicant: Pharmasan Labs, Inc., Osceola, WI 54020 (US)
(72) Inventor: Scholz, Felix, Saint Paul, MN 55106 (US); Kellermann, Gottfried, Osceola, WI 54020 (US)
(74) Representative: Müller-Boré & Partner Patentanwälte PartG mbB

(57) **Abstract**

The present invention relates to methods for the qualitative and quantitative determination of the allergic status of a subject in relation to one or more allergens of interest, comprising steps of determining IL-4 and/or histamine secretion of basophils in response to stimulation with said allergens. The present invention further relates to kits for performing said methods.

## Description

The present invention relates to methods for the qualitative and quantitative determination of the allergic status of a subject in relation to one or more allergens of interest, comprising steps of determining IL-4 and/or histamine secretion of basophils in response to stimulation with said allergens. The present invention further relates to kits for performing said methods.

Allergies within the western world civilizations are on the rise. Current diagnosis is relying on skin prick tests and serum IgE measurements. The problem with these current test is that crude allergen extracts are used and super-antigens can cause false positive and false negative responses. In addition, the presence of IgE antibodies in the serum of patients is merely a measure of sensitization and not of allergy. In particular, patients with IgE antibodies can nevertheless be non-responders. Furthermore, the standard procedure to measure the efficacy of immunotherapy is a re-challenge of allergic patients which is potentially harmful and/or inconvenient for the patient.

Basophils are a small subset of immune cells within the peripheral blood mononuclear cell (PBMC) population of the blood. They bind specific IgE antibodies on their cell surface via the receptor FcεRI and get activated by specific antigens/allergens. They release histamine and cytokines such as IL-4 after activation. Accordingly, basophils are among the main mediators of symptomatically and clinically manifest allergy.

In view of the above, the technical problem underlying the present invention is the provision of means for the reliable, specific, easy, and safe diagnosis of symptomatically and clinically manifest allergy.

The solution to the above technical problem is achieved by the embodiments characterized in the claims.

In particular, in a first aspect, the present invention relates to a method for the qualitative and quantitative determination of the allergic status of a subject in relation to one or more allergens of interest, said method comprising the steps of:
(a) providing peripheral blood mononuclear cells (PBMCs) from the subject;
(b) providing a surface having one or more discrete wells, wherein said wells are coated with at least one of
   (i) antibodies, antibody fragments, or antibody mimetics directed against IL-4, and
   (ii) antibodies, antibody fragments, or antibody mimetics directed against histamine;
(c) plating said PBMCs in said one or more wells;
(d) adding IL-3 to said one or more wells;
(e) adding said one or more allergens of interest to said one or more wells;
(f) incubating the surface for a time and at conditions that allow for the secretion of IL-4, and histamine by basophils; and
(g) detecting spots in said one or more wells where IL-4 and/or histamine secreted by basophils has been captured by the respective antibodies, antibody fragments, or antibody mimetics;
wherein each detected spot represents a basophil that responded to the allergen present in the respective well with the secretion of IL-4, and/or histamine, respectively, and
wherein the amount of spots correlates with the severity of the subject's allergic reaction.

In this context, the term "qualitative and quantitative determination of the allergic status of a subject" as used herein relates to the fact that with the methods of the present invention, not only the presence or absence of an allergy against a given allergen, but also the severity of the allergic reaction can be determined. Further, the term "allergic status of a subject" as used herein relates to both of these qualitative and quantitative parameters, *i.e.,* said term encompasses information concerning the presence of an allergy against a given allergen, as well as the severity of the respective allergic reaction.

Allergens of interest that can be used and assessed in the methods of the present invention are not particularly limited and include any compounds, compositions or substances that are known to or suspected of causing allergic reactions. Allergens can be native isolated allergens such as allergen extracts, or recombinant allergens. Allergens can be selected from the group consisting of pollen allergens, drug allergens, environmental allergens, animal allergens, and food allergens. Exemplary environmental allergens include mold or dust mite allergens. Exemplary animal allergens include cat and dog allergens. Exemplary food allergens include allergens derived from peanut (e.g. ara h 1, 2, 3, 4, 5, 6), milk, soy, shell fish, fish, meat, and tree nuts. Exemplary animal toxins include insect venoms.

According to step (a) of the methods of the present invention, peripheral blood mononuclear cells (PBMCs) are provided from the subject. This step expressly excludes the step of obtaining a respective sample, e.g. a whole blood sample, from the patient. PBMCs can be isolated from whole blood or other sample material by methods known in the art, e.g. by density gradient centrifugation.

The surface having one or more discrete wells provided in step (b) of the methods of the present invention can be any well plate known and/or used in the art. Preferably, said plate is a multi-well plate, e.g. a 96-well plate. However, any other multi-well format can equally be used.

According to the present invention, the wells of said surface are coated with at least one of (i) antibodies, antibody fragments, or antibody mimetics directed against IL-4, and (ii) antibodies, antibody fragments, or antibody mimetics directed against histamine. In this context, the term "antibody fragments" as used herein relates to Fab fragments, F(ab')₂ fragments and Fab' fragments. Further, the term "antibody mimetic" as used herein relates to proteinaceous compounds that, like antibodies, are capable of binding to a given target structure in a specific manner. Preferably, antibody mimetics are selected from the group consisting of single-chain variable fragments (scFv), single-domain antibodies, affibodies, affilins, affimers, affitins, anticalins, DARPins, monobodies, and peptide aptamers.

Methods for the coating of a surface, e.g. the wells of a multi-well plate, with antibodies, antibody fragments, or antibody mimetics are not particularly limited and are known in the art. They include for example the incubation of said surface with a respective antibody, antibody fragment, or antibody mimetic solution, e.g. at 4°C for at least 12 hours.

In this context, the term "directed against (...)" as used herein indicates the antigen that is specifically recognized by the respective antibody, antibody fragment, or antibody mimetic. By way of example, the term "antibody directed against IL-4" is expressly used synonymously to the term "anti-IL-4 antibody".

It should be noted that the methods of the present invention, when used in a multi-well format, allow for each well the specific selection of antibody coating, type of coating antibody, addition of cells, amount of cells added, addition of IL-3, amount of IL-3 added, addition of allergens, type of allergens, amount of allergens added, and addition of negative or positive control solutions, as suited for the respective assay to be conducted. Thus, the terms "said wells are coated", "adding (...) to said wells", and the like are expressly intended to relate to the fact that only selected wells as suitable for the assay to be conducted are meant. By way of example, a multi-well plate used in a method of the present invention may include wells that are not coated, that do not receive any cells, that do not receive any IL-3, that do not receive any allergens, that receive positive or negative control solutions, and/or that do not receive any detection agents.

In step (c) of the methods of the present invention, PBMCs are plated in the one or more wells. The number of cells per well is suitably selected and may be between 10³ and 10⁶ cells, preferably about 250000 cells.

In step (d) of the methods of the present invention, IL-3, preferably recombinant human IL-3 (hrIL-3) is added to the wells, preferably in a concentration of 10 to 100 ng/ml, more preferably 10 to 50 ng/ml, e.g. about 30 ng/ml. IL-3 serves as a growth factor for basophils, allowing the growth of said cells.

In step (e) of the methods of the present invention, the one or more allergens of interest are added to the one or more wells. In this context, it is preferable that one allergen is added per well, although combinations of allergens can also be used in one well. The amount of allergen can be between 0.01 and 50 µg/ml, preferably between 0.01 and 30 µg/ml. Further, it can be reasonable to use dilution series of allergen in different wells, e.g. 0.15, 1.5, and 15 µg/ml.

In this context, it should be noted that steps (c), (d), and (e) can be performed simultaneously, e.g. the PBMCs, IL-3 and allergens can be combined prior to addition to the wells.

Step (e) of the methods of the present invention can further comprise the step of adding negative and/or positive controls to selected wells. As negative control, medium can be used, e.g. the medium in which also the allergens are solved. As positive control for basophils, anti-Fcε antibodies can be used in combination with the peptide fMLP (*N*-Formylmethionine-leucyl-phenylalanine).

Step (f) of the methods of the present invention encompasses incubation of the surface for a time and at conditions that allow for the secretion of IL-4, and histamine by basophils. Suitable conditions and durations are known in the art. For example, the surface can be incubated at 37°C for at least 12 hours.

In step (g) of the methods of the present invention spots in said one or more wells where IL-4 and/or histamine secreted by basophils has been captured by the respective antibodies, antibody fragments, or antibody mimetics are detected. Respective methods for detecting said spots are not particularly limited and are known in the art.

Preferably, step (g) of the methods of the present invention comprises the steps of:
(g1) adding detection antibodies, antibody fragments, or antibody mimetics directed against IL-4 and/or detection antibodies, antibody fragments, or antibody mimetics directed against histamine to said one or more wells, said detection antibodies, antibody fragments, or antibody mimetics having a binding moiety;
(g2) adding a secondary detection agent to said one or more wells, said secondary detection agent being capable of binding to said binding moiety, and having a signal-generating moiety; and
(g3) detecting the signal generated by said signal-generating moiety.

In this embodiment, the respective detection antibodies, antibody fragments, or antibody mimetics are preferably directed against a different epitope of the respective antigen as the antibodies, antibody fragments, or antibody mimetics used for coating. The binding moiety can be any structure or compound that can be specifically recognized by a binding partner, *i.e*., by the secondary detection agent. Suitable binding pairs are not particularly limited and are known in the art. Further, the signal-generating moiety can be any structure or compound that can directly or indirectly generate a detectable signal. In preferred embodiments, said binding moiety is biotin, said secondary detection agent is streptavidin, and said signal-generating moiety is alkaline phosphatase (AP) or horseradish peroxidase (HRP), wherein the methods of the present invention further comprise, after step (g2) and prior to step (g3), a step of adding a suitable detection solution to said one or more wells. Suitable detection solutions for use in combination with AP or HRP are not particularly limited and are known in the art. In other embodiments, the signal-generating moiety is a fluorescent moiety, such as e.g. a fluorescent dye as known in the art.

In the methods of the present invention, the amount of spots correlates with the severity of the subject's allergic reaction, *i.e*., the more spots are detected, the more severe the subject's allergic reaction to the respective allergen is.

In a preferred embodiment, the methods of the present invention further comprise, after step (a) and prior to step (c), a step of depleting T cells from said PBMCs. Respective methods for depleting T cells from PBMCs are not particularly limited and are known in the art. In this context, depletion of T cells can be employed to ensure that a basophil response is detected. However, even in the presence of T cells, spots detected in the methods of the present invention are to a vast majority derived from basophils. Therefore, T cell depletion is not a necessity.

Preferably, the subject in the methods of the present invention is human.

In a second aspect, the present invention relates to a kit, comprising the surface, antibodies, antibody fragments, or antibody mimetics, detection antibodies, antibody fragments, or antibody mimetics, secondary detection agents, and optionally detection solutions as defined.

As used herein, the term "about" is intended to be a modifier of ± 10% of the specified value. As an example, the term "about 250000" is intended to encompass the range of 225000 to 275000.

The terms "comprising/comprises", "consisting of/consists of", and "consisting essentially of/consists essentially of" are used herein in an interchangeable manner, *i.e*., each of said terms can expressly be exchanged against one of the other two terms.

The allergy screening panel based on allergen-specific Basophil responses according to the present invention is a novel platform of a cell-based assay to screen for allergies in whole blood samples of patients. It is based on the principle that basophils respond with Histamine and IL-4 release after encountering specific allergenic epitopes. The allergy screening panel is designed in a multi-well format. When incubated with recombinant allergen epitopes, only basophils from allergic patients will respond and release Histamine and IL-4 and the released molecules can be visualized as spots on a membrane where one spot represents one cell that responded. This novel basophil assay will simplify screening for specific allergen epitopes and will aid to tailor a patient specific desensitization treatment. Furthermore, the amount of spots correlates with the severity of an allergy and, therefore, helps to predict how strong a patient will respond after being exposed to allergens.

The conjunction of allergen specific basophil responses in 96-well format to screen for allergen specific immune responses provides a novel platform to assess the allergic state of a patient. This test provides enhanced sensitivity over Serum IgE or skin prick testing. The principle of this invention is measuring IL-4 release as a measure of cellular response to cognate allergen binding specific IgE antibodies and crosslinking FcεRI on basophils. The activation of basophils is depending on the amount of surface bound allergen and specific IgE. Measuring the T cell frequency and specific amounts of serum level IgE alone is not reflective of the allergic status of a patients as sensitized persons that are non-responders display allergen specific T-cells and might have certain levels of specific serum IgE present. Measuring the number of activated basophils that have released IL-4 and/or histamine provides a superior tool in comparison to the currently approved methods for allergy measurements. Furthermore, the multiwell plate format of the present invention provides a novel approach for health care providers to screen for specific allergic epitopes and asses to which epitopes the patients are responding in a convenient and simple format. The data gained by this test can be used to tailor a patient specific treatment plan for immunotherapy. Furthermore, it gives health care providers an assessment tool at hand to monitor the allergic responses throughout immunotherapy without using current state of the art methods such as oral food challenge that might harm the patient.

IL-3 as a growth and survival factor for basophils is enabling the measurement of specific IgE responses and sensitization status of the patient. Although IL-3 can induce IL-4 production in basophils at the levels used for this test, this phenomenon can be easily controlled for.

Taken together, the allergy analysis platform according to the present invention is intended as a novel tool to screen for allergy in general, screen for specific allergenic epitopes, to assess the allergic status of a patient and to monitor allergic responses during immunotherapy.

Basophil antigen/allergen specific responses provide a novel screening format for allergies. Patient derived basophils can be tested with multiple panels of allergens in a 96-well format. This can be utilized with allergen extracts or specific recombinant allergens to identify specific epitopes a patient is responding to. It is suitable for all allergens, such as environmental allergens, pollen, insect venom, medication allergies and food allergies, as true allergies are all IgE mast cell/basophil dependent immune responses.

The methods of the present invention will be a helpful tool for a health care provider to understand and diagnose the allergic status of a patient. Especially during desensitization treatment it provides with a simple blood draw a superior and less dangerous way to measure treatment efficacy compared to allergen challenge of the patient.

The figures show:
Figure 1:
   Basophil allergen test.
   Negative control, anti IgE positive control and Fel d 1 (15 ng/ml) stimulated PBMCs of a cat allergic patient. Negative control displays low spot counts, Anti-IgE basophil positive control displays basophil specific spots and PMBCs stimulated with cat specific allergen fel d 1 display high spot counts.
Figure 2:
   Basophil responses enhance sensitivity.
   PBMCs of cat allergic patients, previously diagnosed by cat specific skin prick test. The cells were incubated in basophil culture medium containing IL-3. Basophil allergen specific response is measure by IL-4 positive spots. Spot counts ranged from 8 to 421 and resulted for 2 of the 3 allergic patients in clearly significant distinct spot counts over negative controls.
Figure 3:
   Increased sensitivity with basophil specific allergen screening method.
   Cat allergic patients were compared with and without basophil screening medium. The spot count was increased after stimulating PBMCs of allergic patients with IL-3 present in the mix.
Figure 4:
   IL-4 spots are basophil specific.
   PBMCs of cat allergic and non-allergic patients were treated split and half of the sample was depleted of basophils with anti-IgE depleting antibody. Total PBMCs and basophil depleted PBMCs were treated with anti-FcεR-I antibody or with cat allergen Fel d 1. Cat allergic patients displayed high IL-4 spot counts while non-atopic persons did not. The IL-4 positive spots were diminished after basophil depletion indicating that the positive IL-4 spots after treatment with anti-FcεR-I antibody or with Fel d 1 originate from basophils.
Figure 5:
   Basophil spot counts correlates with CD63 up-regulation of basophils.
   The basophil activation test measures the cell surface marker CD63 as a measure of basophil activation. It has been shown that basophil activation and up-regulation of CD63 correlates with the allergic status of a patient. Correlation comparison with this method shows that basophil spot counts are correlating with CD63 surface expression after allergen exposure.
Figure 6:
   Basophil spot count is applicable for allergen testing.
   Allergic reactions are based on the principle that allergen specific IgE is bound to surface expressed FcεRI on basophils and mast cells. Exposure to the allergen leads to degranulation and Histamine and IL-4 release. This universal allergy principal allows for the screening and detection of all allergies as shown in this figure for the cat allergens Fel d-1 and the peanut allergens Ara h-2 and Ara h-6. The allergic patients displayed spot counts of 72 to 535 spots, depending on the specific allergy and allergic status of the patient. All patients have been diagnosed by skin prick test for their respective allergy.
Figure 7:
   Basophil responses are specific.
   Exposure to non-relevant epitopes does not induce basophil activation in this assay. PBMCs obtained from cat allergic patients were exposed to the peanut allergen Ara h 2. The observed spot count was comparable to unstimulated PBMCs. Similar results were observed when PBMC from Peanut allergic persons were exposed to the major soy allergens Gly m 4 or Gly m 5.

The present invention will be further illustrated by the following examples without being limited thereto.

### Examples

### Methods:

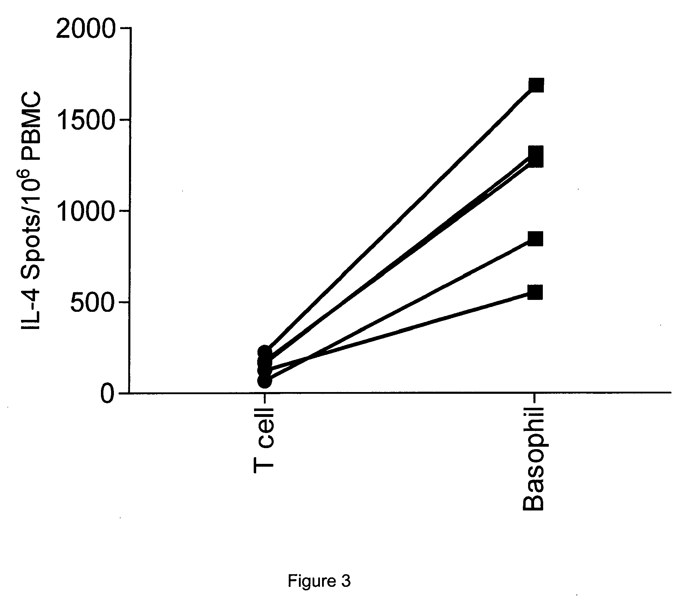

Donors with and without diagnosed allergies for cat and peanut where picked and blood was collected in ACD tubes. Donors that qualified for allergic were skin prick positive and displayed Serum IgE for a specific allergy (cat, peanut, etc.). All experimental procedures where according to the proposed invention. In brief: PMBCs were isolated with density centrifugation and plated at 250000 cells per well. In case of the basophil depletion experiment, an anti-IgE antibody that crosslinks basophils to red blood cells was used to pull basophils out of the sample. Equal amounts of total cells where plated and analyzed (Figure 3). The cells were incubated in presence of IL-3 with recombinant allergens.

The results indicated that when basophils are present IL-4 positive spots can be measured if a sample is treated with either FcεR-I, a basophil specific activator, or if PBMCs of an allergic donor are incubated with the relevant epitope. Furthermore, these spots are not present if the basophil population was depleted from the patient samples, indicating that basophils are the major producers of IL-4 under the suggested experimental conditions.

Depending on the set up of the experiments, PBMCs of allergic and healthy donors where treated with anti FcεR-I and fMLP as positive control for basophils. The wells indicated that basophils are responding well under suggested experimental conditions. Furthermore, PBMCs of donors where incubated with relevant or non-relevant allergic epitopes to explore the possibility of false positivity or cross reactivity. Non relevant epitopes result in some back ground spots but relevant epitopes display a significant higher spot formation.

For method comparison, the collected blood of allergic donors was run in comparison with the basophil activation test. The test is performed with fluorescent staining of basophils and CD63 as a surrogate marker for Histamine release. The results indicate that both test correlate with each other

### Example 1:

PBMC's from allergic patients respond to exposure of a relevant recombinant allergen with production of IL-4.

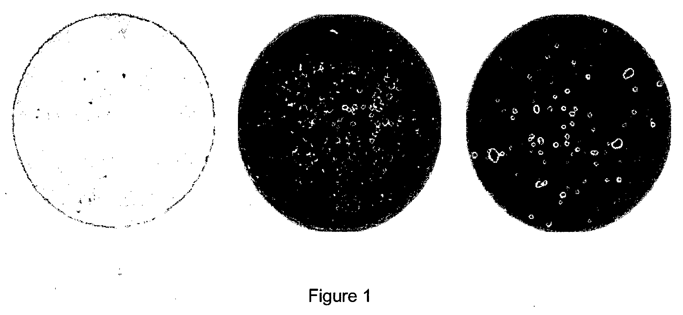

Allergic immune responses are characterized by a TH2 response. T cells that are sensitized for specific allergens produce upon re-stimulation with the cognate antigen TH2 cytokines. They provide T cell help and induce in B-cells a class switch to IgE antibodies. These antibodies are bound to the high affinity receptor FC-εR-I on the surface of mast cells within epithelial tissues or in the surface of basophils within the blood. Mast cells and basophils respond upon antigen recognition with release of Histamine that induces the allergic response. Basophils produce IL-4 and IL-13 after the release of histamine. PBMCs of cat allergic donors respond with IL-4 production after exposure to recombinant Feld 1 (Figure.1). Also anti IgE antibodies that specifically activate basophils induce IL-4 expression in a subset of cells within the PBMCs, while the negative control remained negative. These results indicate that basophils are producing IL-4 in response to specific stimuli such as anti IgE antibodies or specific recombinant allergens.

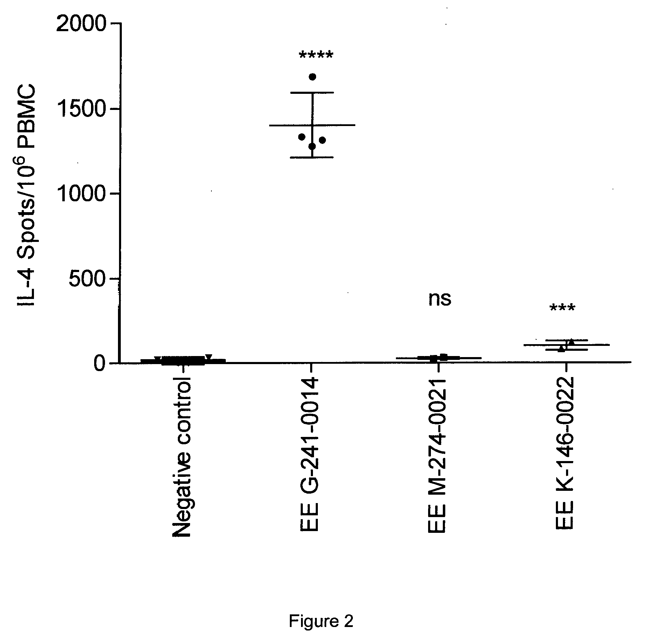

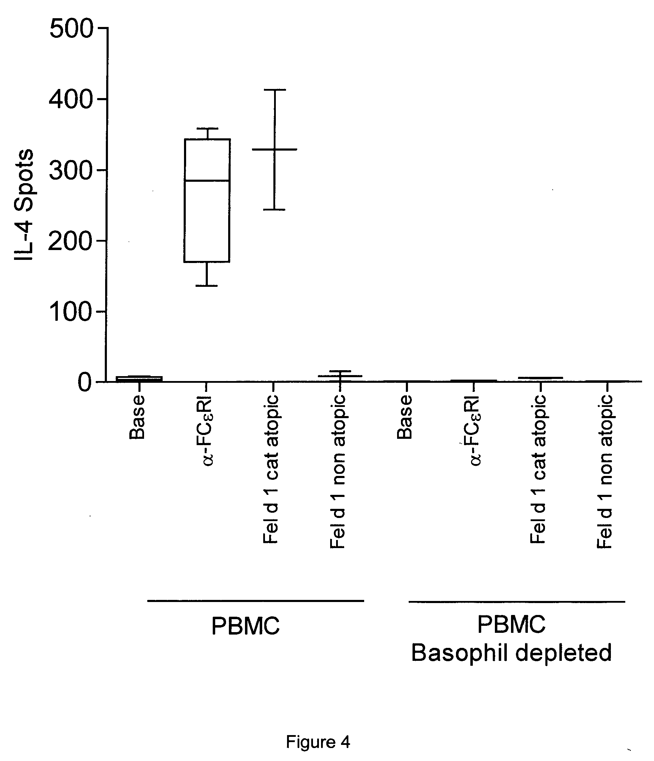

The observed IL-4 spot formation was depending on the donor and was constant over time. Three different cat allergic donors where analyzed multiple weeks in a row. Two donors displayed significant spot counts compared to the negative controls, while one donor did not display significant higher spot counts (Figure 2). The amount of IL-4 producing cell stayed constant over time. Later experiments confirmed that the patient with barely any IL-4 positive spots was tested as a low responder in the basophil activation test (Figure 4).

### Example 2:

IL-4 spots are depending on basophils.

IL-3 is a crucial survival factor for basophils. IL-3 was added to the medium to enhance basophil survival and enable to measure basophil specific responses to recombinant allergens. Comparison of IL-4 spot formation from cat allergic donors with and without IL-3 showed a significant increase in spot counts (Figure 3)

To asses if the spot increase was due to basophil survival and activity, basophils were depleted from the PBMC subsets of healthy and allergic donors. Basophil depletion with an antibody against IgE that crosslinks basophils to red blood cells during the density centrifugation step was used. Whole blood of cat allergic and non-allergic donors was spitted in halve and one part was not treated and the other was incubated with an antibody against IgE that crosslinks basophils to red blood cells. During density centrifugation the red blood cells and the linked basophils get depleted from the sample, leaving a PBMC population behind that lacks basophils. Untreated PBMCs that were incubated with Fel d 1 produced IL-4 spots if they were obtained from cat allergic donors. PBMCs of cat allergic donors that were depleted of basophils failed to form IL-4 positive spots. This was also true for the incubation of basophil depleted PBMCs with the specific basophil activator anti-IgE antibody, indicating that in the proposed assay format the IL-4 producing cells are basophils (Figure 4).

### Example 3:

Basophil IL-4 spot formation correlates with basophil activation.

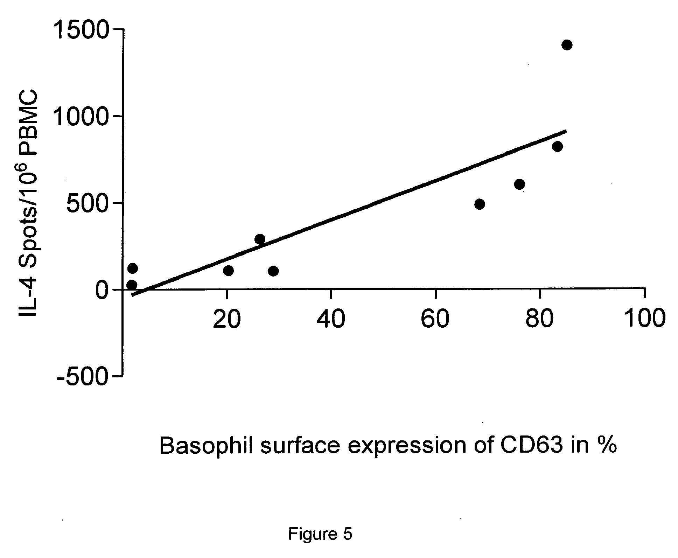

For method comparison blood of cat allergic donors was collected and run with the established basophil activation test and the IL-4 basophil iSpot in comparison. The basophil activation test is a highly specific test that utilizes extracellular staining for CD63. This membrane bound molecule is present in histamine vesicles and gets shuttled to the cell surfaces of basophils during activation after allergen exposure. The surface expression of CD63 correlates with the severity of an allergic donor. The method comparison showed that the IL-4 spot formation correlated well with the CD63 surface expression on basophils. Donors that displayed low levels of surface CD63 expression displayed low spot formation. And donors that displayed high basophil activation displayed high spot formation (Figure 5). These results indicate that the IL-4 spot formation correlates with the severity of an allergy present in a donor.

### Example 4:

Basophil IL-4 spot formation is allergen specific response.

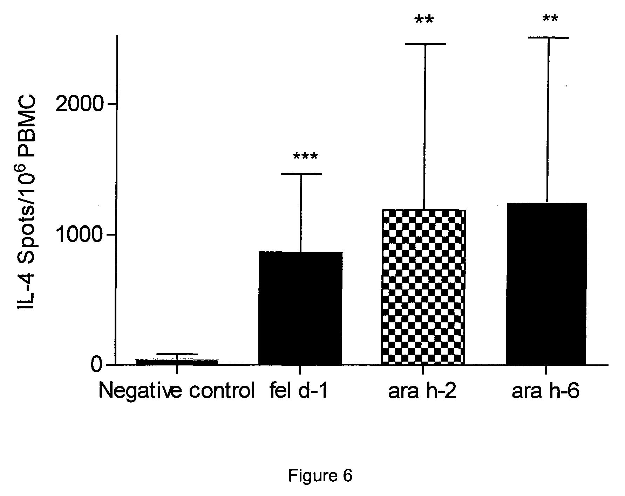

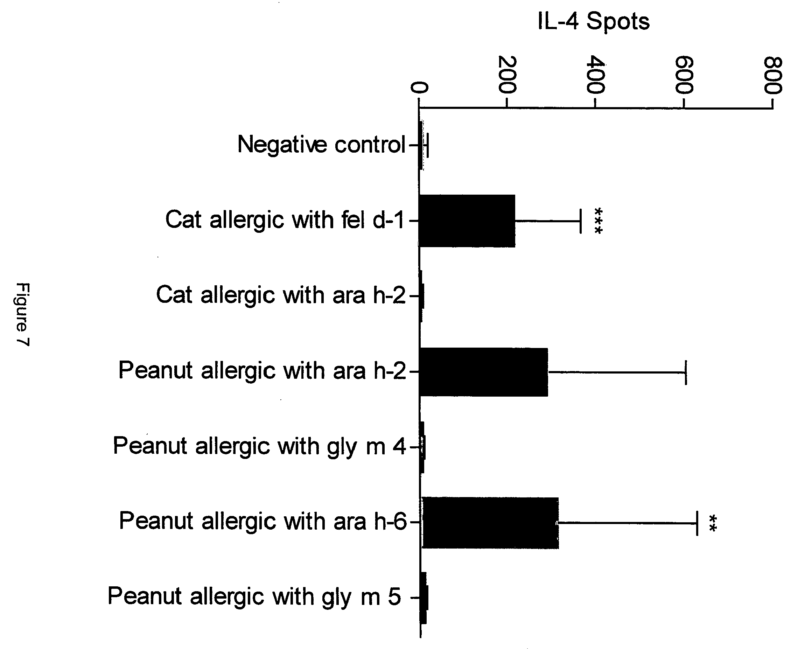

The suggested assay format is suitable for all types of IgE mediated allergies. Cat and Peanut allergic donors have been tested with specific recombinant allergens and displayed significant spot count compared to negative controls (Figure 6). These responses are specific as basophils from cat allergic donors responded with IL-4 production to exposure of Fel d 1 but did not produce IL-4 positive spots in response to peanut allergens such as Ara h 2. PBMCs obtained from peanut allergic donors responded well to Ara h 2 and Ara h 6 exposure but did not produce IL-4 in response to soy allergens Gly m 4 and Gly m 5 (Figure 7). These results show that the assay is specific for the allergies that are present in a donor and is able to distinguish between sensitized persons and not sensitized persons.

### Example 5:

The following details an exemplary protocol for performing the methods of the present invention.
- Coat 96 wells with antibody (either directed against IL-4 or histamine)
   ∘ Pre-wet membrane with 70% EtOH for less than 60s
   ∘ Wash 3x with PBS
   ∘ Dilute coating antibody in appropriate concentration
   ∘ Plate 80 µl of antibody solution per well
- Incubate at 4°C over night
- Provide blood in an ACD or EDTA tube
- Store/ship on 4°C
- Overlay Ficoll gradient with whole blood
- Centrifuge for 30 min at 2500 rpm 50% breaks
- Carefully pipet cell layer into 50ml Falcon tube
- Wash 2 times with PBS and centrifuge at 250g 10 minutes
- Count cells
- Prepare Basophil Medium with 1% fresh L-glutamine and 30ng/ml hrIL-3 (recombinant human IL-3)
- Plate 250000 cells per well
- Add medium for negative control, and anti-Fc-ε and fMLP for basophil positive control
- Add recombinant allergens to wells at 0.15, 1.5 and 15 µg/ml final concentration
- Incubate at 37°C over night
- Wash 2x with PBS
- Wash 2 x with PBS 0.05% Tween
- Dilute biotinylated detection antibody 1:1000 in PBS tween (anti-IL-4 or antihistamine)
- Plate 80 µl per well
- Incubate 2h at room temperature (RT)
- Wash 4 times with PBS Tween
- Dilute AP- or HRP-streptavidin conjugate at 1:1000
- Plate 80 µl per well
- Incubate 30 minutes at RT
- Wash 2x with PBS Tween
- Wash 2x with distilled water
- Prepare detection solution
- Plate 80 µl per well
- Incubate for 15 minutes at RT
- Wash with water to stop biochemical reaction
- Dry plate
- Measure plate
- Enumerate Spots

## Claims

1. A method for the qualitative and quantitative determination of the allergic status of a subject in relation to one or more allergens of interest, said method comprising the steps of:
(a) providing peripheral blood mononuclear cells (PBMCs) from the subject;
(b) providing a surface having one or more discrete wells, wherein said wells are coated with at least one of
(i) antibodies, antibody fragments, or antibody mimetics directed against IL-4, and
(ii) antibodies, antibody fragments, or antibody mimetics directed against histamine;
(c) plating said PBMCs in said one or more wells;
(d) adding IL-3 to said one or more wells;
(e) adding said one or more allergens of interest to said one or more wells;
(f) incubating the surface for a time and at conditions that allow for the secretion of IL-4 and histamine by basophils; and
(g) detecting spots in said one or more wells where IL-4 and/or histamine secreted by basophils has been captured by the respective antibodies, antibody fragments, or antibody mimetics;
wherein each detected spot represents a basophil that responded to the allergen present in the respective well with the secretion of IL-4 and/or histamine, respectively, and
wherein the amount of spots correlates with the severity of the subject's allergic reaction.

2. The method of claim 1, further comprising, after step (a) and prior to step (c), a step of depleting T cells from said PBMCs.

3. The method of claim 1 or claim 2, wherein said one or more allergens are recombinant allergens.

4. The method of any one of claims 1 to 3, wherein said one or more allergens are selected from the group consisting of pollen allergens, drug allergens, environmental allergens, animal allergens, and food allergens.

5. The method of any one of claims 1 to 4, wherein said surface is a multi-well plate.

6. The method of any one of claims 1 to 5, wherein step (f) comprises incubation at 37°C for at least 12 hours.

7. The method of any one of claims 1 to 6, wherein step (g) comprises the steps of:
(g1) adding detection antibodies, antibody fragments, or antibody mimetics directed against IL-4 and/or detection antibodies, antibody fragments, or antibody mimetics directed against histamine to said one or more wells, said detection antibodies, antibody fragments, or antibody mimetics having a binding moiety;
(g2) adding a secondary detection agent to said one or more wells, said secondary detection agent being capable of binding to said binding moiety, and having a signal-generating moiety; and
(g3) detecting the signal generated by said signal-generating moiety.

8. The method of claim 7, wherein said binding moiety is biotin, said secondary detection agent is streptavidin, and said signal-generating moiety is alkaline phosphatase or horseradish peroxidase, said method further comprising, after step (g2) and prior to step (g3), a step of adding a suitable detection solution to said one or more wells.

9. The method of any one of claims 1 to 8, wherein step (e) further comprises the step of adding negative and/or positive control solutions to selected wells.

10. The method of any one of claims 1 to 9, wherein the subject is human.

11. A kit comprising the surface, antibodies, antibody fragments, or antibody mimetics, detection antibodies, antibody fragments, or antibody mimetics, secondary detection agents, and optionally detection solutions as defined in any one of claims 1 to 10.
